(19) **Europäisches Patentamt / European Patent Office / Office européen des brevets**

(11) **EP 1 594 571 B1**

(12) **EUROPEAN PATENT SPECIFICATION**

(45) Date of publication and mention
of the grant of the patent:
**30.11.2011 Bulletin 2011/48**

(51) Int Cl.:
***A61N 2/10*** (2006.01)

(21) Application number: **04704151.2**

(22) Date of filing: **22.01.2004**

(86) International application number:
**PCT/AU2004/000089**

(87) International publication number:
**WO 2004/064921 (05.08.2004 Gazette 2004/32)**

(54) **MICROPARTICLES FOR SELECTIVELY TARGETED HYPERTHERMIA**

MIKROTEILCHEN FÜR DIE SELEKTIVE GEZIELTE HYPERTHERMIE

MICROPARTICULES POUR HYPERTHERMIE CIBLEE DE MANIERE SELECTIVE

(84) Designated Contracting States:
**AT BE BG CH CY CZ DE DK EE ES FI FR GB GR HU IE IT LI LU MC NL PT RO SE SI SK TR**

(30) Priority: **22.01.2003 AU 2003900335**

(43) Date of publication of application:
**16.11.2005 Bulletin 2005/46**

(73) Proprietor: **SIRTEX MEDICAL LIMITED**
**North Sydney, NSW 2060 (AU)**

(72) Inventors:
• **JONES, Stephen, Keith**
**North Curl Curl, New South Wales 2099 (AU)**
• **RUTHERFORD, Katrina, Francis**
**Dundas Valley, New South Wales 2117 (AU)**
• **RUYS, Andrew, John**
**Pymble, New South Wales 2073 (AU)**
• **GRAY, Bruce, Nathaniel**
**Claremont, Western Australia 6010 (AU)**

(74) Representative: **Williams, Aylsa**
**D Young & Co LLP**
**120 Holborn**
**London EC1N 2DY (GB)**

(56) References cited:
EP-A- 0 913 167          EP-B1- 1 175 237
WO-A-01/37721           WO-A-97/43005
US-A- 5 427 767          US-A- 6 048 515
US-A1- 2002 027 262     US-A1- 2003 168 640
US-B1- 6 541 039

• DATABASE WPI Week 199806, Derwent Publications Ltd., London, GB; Class A96, AN 1998-053288, XP008093296 & DE 196 24 426 A1 (BREGEMANN) 02 January 1998
• JORDAN ANDREAS ET AL.: 'Magnetic fluid hyperthermia (MFH):cancer treatment with AC magnetic field induced excitation of biocompatible superparamagnetic nanoparticles' JOURNAL OF MAGNETISM AND MAGNETIC MATERIALS vol. 201, 1999, pages 413 - 419, XP004181289
• POULIQUEN DANIEL: 'Magnetite-dextran nanocapsules : preparation and properties' RADIOLABELED AND MAGNETIC PARTICULATES IN MEDICINE & BIOLOGY, MICROSPHERES, MICROCAPSULES & LIPOSOMES vol. 3, 2001, pages 495 - 523, XP008095039

## Description

### Field of the Invention

The present invention relates to microparticle composition(s) capable of heating an object or a target site in appropriate magnetic field conditions. In particular, the microparticle composition(s) are suitable for inducing hyperthermia in tissue. The compositions of the invention are particularly suitable for providing hyperthermic treatment of tissue such as diseased tissue. Further, the present invention provides a method of heating a non-living object as well as a method for manufacturing a medicament suitable for use in treating diseased tissue such as malignant tissue.

### Background Art

The following discussion provides the background to one aspect of the potential uses of the present invention. This discussion should not be read as a limitation on the possible uses to which the present invention may be put. In this respect it should be appreciated that the microparticle compositions of the invention may be used to heat any object provided the microparticles can be arranged in a manner that facilitates heating of the object via the heating effects of the microparticles when placed in a suitable magnetic field.

Diseases of the human body such as malignant tumours are generally treated by excision, chemotherapy, radiotherapy or a combination of these approaches. Each of these is subject to limitations which effects clinical utility. Excision may not be appropriate where the disease presents as a diffuse mass or is in a surgically inoperable locality. Chemotherapeutic agents are generally non-specific, thus resulting in the death of normal and diseased cells. As with chemotherapy, radiotherapy is also non-specific and results in the death of normal tissues exposed to ionizing radiation. Furthermore, some diseases such as tumours may be relatively resistant to ionizing radiation. This is a particular, problem with the core of a tumour mass.

As an alternative, hyperthermia has been proposed as a treatment of diseased tissue. There is evidence to suggest that hyperthermia is effective in treating diseases, including cancerous growths. The therapeutic benefit of hyperthermia therapy is mediated through two principal mechanisms. Firstly, hyperthermia therapy has a direct tumouricidal effect on tissue by raising temperatures to greater than 42°C resulting in irreversible damage to cancer cells. Secondly, hyperthermia is known to sensitise cancer cells to the effects of radiation therapy and to certain chemotherapeutic drugs. The lack of any cumulative toxicity associated with hyperthermia therapy, in contrast to radiotherapy or chemotherapy, is further justification for seeking to develop improved systems for hyperthermia therapy.

Several techniques are presently available for inducing clinical hyperthermia either regionally, in selected local regions of specific organs or over the whole body. These techniques include the use of plain wave electromagnetic radiation, such as microwave heating and radiofrequency waves, and ultrasound.

The above techniques have their limitations, including poor tissue penetration and a rapid decline of energy with increasing depth; perturbations induced by tissue interfaces such as air and bone; variation in the heating effect or focusing for deep organ heating.

In addition, there are other problems associated with the currently available methods for inducing clinical hyperthermia in human patients. Normal body tissues and organs are heat sensitive and at temperatures of greater than 42°C many tissues will undergo irreversible damage. In addition, the methods currently available for delivering clinical hyperthermia are non-specific; that is, heat normal tissues as well as tumour cells.

For hyperthermia treatment to be effective, there are two basic requirements. Firstly, there is a need to localise the treatment to the target site and secondly, there is a need to maximise heating within the target site while maintaining hyperthermia therapy within safe operating limits for the patient.

One proposed solution when using hyperthermia therapy involves the use of small magnetic particles that can be heated by applying a high frequency alternating magnetic field. The particles may then be delivered to the target site in a variety of ways, e.g. direct injection, antibody targeting or intravascular infusion. An alternating magnetic field is then applied and heat from the particles causes the tumour temperature to rise above the therapeutic threshold of 42°C. The magnetic field conditions must be such as to cause no interaction with tissue, only with the magnetic particles. In this way, only tissue containing a concentration of the magnetic particles will be heated, irrespective of its location in the body.

In general, the small magnetic particles are iron oxide particles covered with a polymer coating, such as dextran. Although such complexes have been used *in vitro*, any *in vivo* use has been limited by the method of injection used and also as a result of the breakdown of the dextran coating within the body. No clinical studies in human patients have yet been attempted using these particles.

Numerous publications describe various potential schemes for a treatment of cancer by induced hyperthermia using the heat generated by small magnetic particles when exposed to an AC magnetic field. These publications are almost entirely based on experimental results showing the production of heat from magnetic particles in a laboratory scenario. Fewer publications show a system working in an animal model and no publications to date present clinical data from actual human patients.

The reasons for this lack of clinical application, despite the obvious potential of the idea, are problems associated with safety, delivery of a therapeutically effective amount of magnetic material to the target site and achieving adequate heating of the target site only. To date, all previously reported systems fail to meet at least one of these requirements.

The major issue with safety is the strength and frequency of the applied magnetic field to be used in heating the magnetic particles. There is currently no way to reliably produce a long narrow beam of magnetic field that only exposes a small area of the patient to the AC field. Hence, systems must be designed assuming the patients' whole body will be exposed to the AC field. This places serious constraints on the amplitude and frequency of the field that can be used since there are potentially hazardous physiological responses to AC fields once the frequency and/or amplitude becomes high enough. These responses include involuntary neuromuscular stimulation and magnetically induced eddy current heating of non-target tissue. In the extreme case, the AC magnetic fields may even cause deadly cardiac arrhythmias.

The heating quality of the complexes including magnetic particles is termed the volumetric absorption rate (VAR) and is defined as the amount of heat released by a unit volume of the complex per unit time during exposure to a magnetic field of a defined frequency and field strength.

To be therapeutically effective, the VAR level should be maintained after the complex has been administered to the patient and have come in contact with biological fluids. For example, the dextran coated particles in the prior art may loose their dextran coatings due to lysosomal enzymatic dextran digestion when used for intracellular MFH (magnetic fluid hyperthermia). This causes particle aggregation and consequent degradation of heating and as such, dextran coated magnetite nanoparticles are not suitable for intracellular MFH.

Many schemes are aimed at ensuring the localised deposition of heat to the cancer tissue, such as direct injection and antibody targeting. Although direct injection may be used to administer high quantities of the magnetic material to a tumour, the technique does have substantial disadvantages, including: (1) tumours must be accurately located and rendered accessible for injection to take place, (2) the physical penetration of tumours can lead to further spread of the cancer, and (3) injected material may not stay in the injected region.

Antibody targeting has not been shown to work effectively, to date. The very small particles of the type required for antibody targeting are also effectively removed from circulation in the blood stream via the reticuloendothelial system. Thus, only short circulation half lives are possible and, hence, limited exposure of the antibody bearing particles to the target cancer cells. At the presently achievable levels of cellular uptake of antibody targeted particles, VAR values far in excess of those presently available would be required to achieve adequate tumour heating even for larger tumours.

Despite the clear advantages of complexes including magnetic particles, there has been no successful utilisation of this methodology in the treatment of human disease to date. This is most likely because of the difficulties faced when scaling up experimental systems to a size suitable for human application. Primary amongst these is the fact that magnetic field conditions (strength and frequency) which might appear safe when used in experiments with small animals, will most likely be unsuitable for use in human patients. However, reduction in the field and or frequency of the AC magnetic field to a level that is safe in human patients invariably leads to a reduction of the VAR of the magnetic material used to heat the tumours to the point where the system becomes useless as a treatment for human patients.

EP-A- 0 913 167 relates to targeted hysteresis hperthermia for treatment of diseased tissue. The document discloses microcapsules containing cobalt treated gamma $Fe_2O_3$ particles as a ferromagnetic material, bound together using a Biopol matrix (a copolymer of (R)-3-hydroxybutyric acid and (R)-3-hydroxyvaleric acid). Using this matrix, magnetic microcapsules in a size range of 20-50 microns can be obtained.

Therefore, there exists a need to develop microparticle composition(s) for heating an object or a target site, when the object or target site is exposed to appropriate magnetic field conditions.

## Summary of the invention

The subject invention relates to microparticle composition(s) capable of heating an object or inducing hyperthermia in a target site when the object or target site is exposed to an alternating magnetic field. The microparticle composition(s) of the invention generate substantial quantities of heat when exposed to a high frequency alternating magnetic field. In one example, the invention relates to microparticle composition(s) for treating diseased tissue by targeted hyperthermia.

According to a first aspect of the invention, there is provided a microparticle composition as defined in claim 1. The composition comprises: nanomagnetic particles and a matrix, wherein said microparticle composition has at least one of the following properties: (a) a VAR of at least about 1 Watts/cm$^3$ subject to appropriate field conditions; and a size of about 100 nm to about 200 microns.

Preferred features of the microparticle composition are defined in the dependent claims.

According to a second aspect of the invention there is provided a method for heating a non-living object including the steps of:

  (i) inserting a microparticle composition(s) as herein described into the object ; and
  (ii) exposing the object to an AC magnetic field wherein the combination of the AC magnetic field with the microparticle compostion induces heat whithin the object.

According to a third aspect, the invention resides in the use of a microparticle composition, as herein described, for the manufacture of a medicament for the treatment of tumours.

**Disclosure of the Invention**

*General*

Those skilled in the art will appreciate that the invention described herein is susceptible to variations and modifications other than those specifically described. It is to be understood that the invention includes all such variations and modifications.

The present invention is not to be limited in scope by the specific embodiments described herein, which are intended for the purpose of exemplification only. Functionally equivalent products, compositions and methods are clearly within the scope of the invention as described herein.

As used herein the term "derived" and "derived from" shall be taken to indicate that a specific integer may be obtained from a particular source albeit not necessarily directly from that source.

Throughout this specification, unless the context requires otherwise, the word "comprise", or variations such as "comprises" or "comprising", will be understood to imply the inclusion of a stated integer or group of integers but not the exclusion of any other integer or group of integers.

Other definitions for selected terms used herein may be found within the detailed description of the invention and apply throughout. Unless otherwise defined, all other scientific and technical terms used herein have the same meaning as commonly understood to one of ordinary skill in the art to which the invention belongs.

*Description*

The present invention relates to a microparticle composition(s) comprising nanomagnetic particles and a matrix. The nanomagnetic particles are incorporated into each microparticle within the matrix and are synthesised to have physical properties that result in a VAR capable of heating an object or target site in appropriate field conditions.

In one example, the microparticle composition(s) is synthesised to have physical properties that result in a maximum VAR in magnetic field conditions that are considered to be clinically safe. More preferably, the resultant VAR is sufficient and suitable for therapeutic heating of diseased tissue, such as cancers.

Although the microparticle composition(s) of the present invention may be used for inducing hyperthermia in tissue, a person skilled in the art would appreciate there are other circumstances where the microparticle composition(s) of the present invention may be used to heat an object or target site.

Microparticle composition(s) of the present invention may be used for heating objects from a diffuse source by an alternating magnetic field. For example, cements or thermoset epoxies may be infused with the microparticle composition(s) of the present invention. The epoxy or cement, once located as desired, may be rapidly heated by application of the magnetic field so that setting occurs rapidly, without heating of the surrounding (non metallic) material. Appropriate field conditions as referred to herein will depend on the nature of the object to be heated and the level of the VAR selected. In the event the microparticles composition(s) are used in human therapy, the VAR will be at least about 10 Watts/cm$^3$ when exposed to the appropriate alternating magnetic field conditions. In a highly preferred embodiment, the VAR is at least about 10 Watts/cm$^3$ and the alternating magnetic field conditions are: a magnetic field strength between about 60 Oe to about 120 Oe and a frequency between about 50 KHz to about 300 KHz.

The foregoing provides merely an illustration of field conditions for human use. When use of the microparticle composition(s) is sought for heating other objects, the appropriate field conditions may well be outside the range described above and which are outside the range considered safe for human therapy. A person skilled in the art will know how to select appropriate field conditions to achieve a suitable VAR for heating other objects.

The heating efficiency of the microparticles produced according to the invention is dictated by a variety of factors. One such factor is the spatial distribution of nanomagnetic particles within the matrix. According to the present invention the nanomagnetic particles are preferably distributed throughout the matrix in a manner which maximizes the heat generated by the microparticles. More preferably, the nanomagnetic particles are distributed throughout the matrix to optimise the simultaneous requirements of maximum particle loading and favourable magnetic interactions between the constituent nanomagnetic particles to achieve maximum VAR for each microparticle.

In a highly preferred embodiment, the nanomagnetic particles are distributed throughout the matrix in such a way as the particles are not in contact with each other.

This may be achieved by dispersing the nanomagnetic particles throughout the matrix so that aggregation of the particles is kept to a minimum during formation of the microparticles. Preferably, only up to 90%, but preferably 80, 70, 60, 50, 40, 30, 20, 10, 5, 1, 0.1, 0.01 or 0.001 % of nanomagnetic particles aggregate in the microparticle. For example, this may be achieved by bonding or attaching a suitable surfactant or coupling agent to the surfaces of the individual nanomagnetic particles prior to their incorporation into the matrix.

When the microparticles within the composition are prepared in such a manner they display at least one or more of the following advantages:

(b) (a) the microparticles are less prone to aggregation and less likely to interact than the complexes known in the prior art; the microparticle composition(s) have a more stable VAR than the complexes of the prior art;

(c) the VAR of the microparticles is more resistant to degradation, particularly when used *in vivo,* as compared to the complexes known in the prior art;

(d) the microparticles are more likely to remain in the correct location for long periods compared to the complexes known in the prior art, thus enabling their repeated use.

The nanomagnetic particles in the microparticle composition(s) of the present invention may be superparamagnetic particles. By the term "superparamagnetic" it is meant magnetic particles that do not have the following properties; (i) coercivity, (ii) remanence or (iii) a hysteresis loop. As such, the VAR arising from the microparticle composition(s) is not a result of hysteresis heating. That is, the physical mechanism that causes heating is "Néel relaxation".

For superparamagnetic particles the VAR is proportional to the quadrature component of the complex susceptibility, i.e. $\chi$". Maximum VAR is obtained when the Néel relaxation time, $\tau_N$, is equal to the inverse of the magnetic field frequency, $\omega$, i.e.

$$\tau_N \omega = 1.$$

In turn, $\tau_N$ is determined by the magnetic anisotropy energy, $KV$, where $K$ is the magnetic anisotropy and $V$ is the particle volume. The value of $K$ is determined by magnetocrystalline anisotropy or the particle shape if it is not perfectly spherical. This assumes particles are smaller than the critical size for formation of magnetic domains, i.e. they are in the superparamagnetic regime.

The properties of VAR, magnetic susceptibility, magnetic moment and saturation magnetization are measurable by standard methods, which can be carried out by those of ordinary skill in the art.

According to the invention the superparamagnetic particles are of nanosize and are preferably selected from within the group of ferrites of general formula $MO.Fe_2O_3$ where M is a bivalent metal such as Fe, Co, Ni, Mn, Be, Mg, Ca, Ba, Sr, Cu, Zn, Pt or mixtures thereof, or magnetoplumbite type oxides of the general formula $MO.6Fe_2O_3$ where M is a large bivalent ion, metallic iron, cobalt or nickel. Additionally, they could be particles of pure Fe, Ni, Cr or Co or oxides of these. Alternatively they could be mixtures of any of these.

In a preferred embodiment, the superparamagnetic particle is a nanoparticle of iron oxide such as magnetite ($Fe_3O_4$) or maghemite ($\gamma$-$Fe_2O_3$) with a particle size preferably less than 50 nanometers and more preferably between 1 and 40 nanometers. In a more highly preferred embodiment, the superparamagnetic particles are maghemite nanoparticles. Such particles are highly preferred because:

(i) maghemite nanoparticles of optimum size possess a higher VAR than do optimum size magnetite nanoparticles when subject to clinically relevant magnetic field conditions, and

(ii) maghemite is a more chemically stable iron oxide than magnetite.

The higher VAR of maghemite means that a lower packing density can be used to produce microparticles with the required VAR.

According to the invention, the ratio of the matrix to the nanomagnetic particles of the microparticle composition(s) can potentially influence the effectiveness of the microparticles, particularly when used *in vivo.* For example, the greater the amount of nanomagnetic particles, the greater the density of the microparticle composition(s). Further, the greater the amount of nanomagnetic particles, the more likely the nanomagnetic particles will clump or aggregate together, thus reducing the VAR of the microparticle composition(s).

In one example, the microparticle composition(s) has a volumetric loading of nanomagnetic particles wherein less than approximately 40% of the microparticle composition(s) is nanomagnetic particles. More preferably, the volume fraction of nanomagnetic particles is less than 30%, but more preferably 20, 15, 10, or 5% of the microparticle composition.

The matrix used in the preparation of the herein described microparticles may be any material known to those skilled in the art. The matrix may display at least one or more of the following advantages:

(a) the matrix enables optimum distribution of the incorporated nanomagnetic particles;

(b) the matrix is relatively light in weight,

(c) the matrix is biocompatible;

(d) the matrix does not interfere with the magnetic characteristics of the nanomagnetic particles.

For example, a polymer matrix may be prepared from any number of different polyurethanes, a styrene divinylbenzene copolymer, ficoll, biopol, chondroitin sulfate, dextran, dextran sulfate, as well as unmodified dextrans. In addition, the matrix may be prepared from a ceramic material. Further, porous silicon and polycrystalline silicon may also be used as matrix material.

According to a preferred embodiment of the invention, the microparticle composition(s) has a volumetric loading of nanomagnetic particles wherein less than approximately 40% of the microparticles composition(s) is nanomagnetic particles and a VAR of at least about 10 Watts of heat per cm$^3$ of their volume (that is a VAR of at least about 10 W/cm$^3$) when exposed to the appropriate alternating magnetic field conditions. In another embodiment, the VAR is 10 Watts/cm$^3$ at magnetic field strength of between 60-120 Oe and frequency between 50-300 KHz. In a highly preferred form of the invention, the VAR is at least 30 W/cm$^3$ in a magnetic field of 60-120 Oe and 50-300 KHz. Preferably, the microparticle composition(s) of the present invention retain their VAR *in vivo*. For example, when the VAR is at least about 10 Watt/cm3, the magnetic field conditions are field strength of 90 Oe and frequency of 100 KHz.

When used in human therapy, the packing density of nanomagnetic particles in the matrix is determined by the need to produce microparticle composition(s) with a sufficiently high level of VAR.

If the required packing density is too high there may be potential problems with the possible use of the microparticles for use in human therapy. For example: (a) if the density of the microparticles is too high it will lead to ineffective transport via the blood stream when introduced *in vivo*, (b) practical difficulties in fabricating microparticle composition(s) with a very high volume fraction of particles, or (c) the heating characteristics of the individual nanomagnetic particles may be degraded due to interaction effects. Therefore, in another preferred embodiment of the present invention, the microparticle composition(s) has a volumetric loading of nanomagnetic particles less than approximately 40% of the microparticle composition(s) and a density less than 2.7 g/cm$^3$. In a highly preferred embodiment, the density is between 2.0 and 2.5 g/cm$^3$.

In an embodiment, the microparticle composition(s) has a volumetric loading of nanomagnetic particles wherein less than approximately 40% of the microparticle composition(s) is nanomagnetic particles and having a size range from about 100 nm to 200 microns, preferably 10 to 50 microns, more preferably 20 to 45 microns and highly preferably from 25 to 37 microns.

In a highly preferred embodiment the microparticle composition(s) of the present invention has a volumetric loading of nanomagnetic particles wherein less than approximately 40% of the microparticle composition (s) is nanomagnetic particles, a VAR of at least about 10 W/cm$^3$ subject to appropriate field conditions, a density of about 2.7 g/cm$^3$ or less and a size of about 20 microns to about 45 microns.

A number of different methods may be used to prepare the microparticles of the present invention using a diverse number of manufacturing techniques. For example, microparticles can be manufactured from a biocompatible polymer using a solvent evaporation technique. The nanomagnetic particles are first dispersed in the dissolved polymer. This solution is then dispersed in water containing a small percentage of PVA and mixed with a high-speed mixer for a few minutes. Finally the mixture is gently stirred for about 1 hour to allow all the solvent to evaporate, leaving the solidified polymer microparticles containing nanomagnetic particles. The microparticles are then sieved to select those in the size range 20-45 micron. These are then separated using a fluid with a high specific gravity (e.g. di-iodomethane appropriately diluted with acetone) to select microparticles of the required density.

For ease of description the following discussion of the invention will focus on the use of the invention in the treatment of diseased tissue. Importantly the skilled reader should understand, however, that the following description is only provided for ease of description of the invention and should not be read as limiting the possible applications for the herein described microparticle compositions. Clearly, as contemplated above, the microparticle composition of the invention has applications beyond the mere treatment of diseased tissue.

According to a preferred embodiment of the invention, there is provided microparticle composition(s), as herein described, which are adapted for use in a patient and which are capable of heating tissue from that patient when exposed to an alternating magnetic field. In one highly preferred use the present invention provides a microparticle composition(s) by which deep seated cancers such as liver cancer can be effectively and safely heated in human patients.

When used to treat human tissue, the microparticle composition(s) of the present invention are preferably of a size which ensures they are capable of being trapped in the capillary bed of tumours rather than being able to pass through the tumour into the venous supply. For example, the microparticles will be approximately 25 to 37 microns with a standard deviation of less than 10%.

The matrix used in the preparation of the microparticles in the microparticle composition(s) may be non-biodegradable or biodegradable. The type of matrix used will thus generally depend on the required life expectancy of the microparticles and the environmental conditions in which the microparticles will be operating. For example, if the microparticle composition were used to treat malignant tissue, the microparticles would need to be relatively non-biodegradable so that they can be used to repeatedly treat the tissue until it has been destroyed. In other situations, however, it might be envisaged that the microparticles would be used to heat a surrounding material and then degrade in a manner which facilitates their removal from the material.

In one preferred embodiment of the invention, the microparticle composition(s) comprise a polymer matrix that is biodegradable, for example polyglycolide (PGA) or chitosan. Suitable polymers are also preferably soluble under their reaction conditions and may include, for example, ficoll, chondroitin sulfate and dextran sulfate, as well as unmodified dextran.

In an alternate embodiment, the matrix used in the preparation of the microparticles is non-biodegradable. Thus, in use, after the initial administration of a microparticle composition(s) to a patient, the microparticles may be repeatedly reactivated as required over a period of time. Therefore, the microparticle composition(s) are capable of acting like an implant that can be controlled at will rather than as a drug or radiopharmaceutical whose action cannot be controlled once administered. By way of example only, the non-biodegradable matrix may be selected from polymers, such as biopol or certain types of polyurethane. Preferably, the polymer matrix is a styrene divinylbenzene copolymer. Other matrix materials include ceramics, such as alumina, zirconia and silica.

Where microparticle compositions are administered to a patient, preferably the microparticles as well as any component of the composition will be biocompatible material. More preferably, the biocompatible material and any other materials used in the preparation of the composition do not contain toxic levels of impurities such as fatty acids and synthetic surfactants. In one example of the invention the microparticle composition will be prepared from non-biodegradable materials.

In accordance with another preferred embodiment of the invention the microparticles in the herein described composition may be adapted for site specific delivery by combining the microparticles with, for example tumour seeking agents, such as tumour-specific antibodies, porphyrins, liposomes, lipoproteins, lectins, or tumour surface receptor-binding agents. Such combinations can be by chemical bond or via physical interaction, such as for example, absorption or lipid bilayer coating. Those skilled in the art will be familiar with the manufacture of such molecules. The microparticles of the present invention may be further associated with agents that would favour their accumulation at the target site. Agents include, by way of example only, ferritin, or RES_masking agents, such as monosialogangliosides or polyethyleneglycol.

Compositions produced according to the present disclosure may simply comprise the microparticles within a fluid medium. Preferably, that medium will be selected based on the end use for the microparticles. For example, if the microparticles were employed to heat a biological sample such as a biological tissue the fluid medium may include, without limitation, at least a carrier, diluent or buffer.

Generally, where the microparticle composition of the invention is employed either in the preparation of a medicament or used in a method to treat diseased tissue, the composition will comprise an amount of microparticles capable of being therapeutically effective when exposed to an AC magnetic field and at least a pharmaceutically acceptable carrier or diluent.

The phrase "pharmaceutically acceptable carrier or diluent" refers to molecular entities and compositions that are physiologically tolerable and do not typically produce an allergic or similarly untoward reaction, such as gastric upset, dizziness and the like, when administered to a human. Preferably, as used herein, the term "pharmaceutically acceptable" means approved by a regulatory agency of the federal or a state government or listed in the *U.S. Pharmacopeia* or other generally recognized pharmacopeia for use in animals, and more particularly humans. The term "carrier" refers to a diluent, preservatives, solubilizers, emulsifiers, excipient, or vehicle with which the microparticles are administered. Suitable pharmaceutical carriers are described in Martin, Remington's Pharmaceutical Sciences, 18th Ed., Mack Publishing Co., Easton, PA, (1990). Such pharmaceutical carriers can be sterile liquids, such as water and oils, including those of petroleum, animal, vegetable or synthetic origin, such as peanut oil, soybean oil, mineral oil, sesame oil and the like. Water or soluble saline solutions and aqueous dextrose and glycerol solutions are preferably employed as carriers, particularly for injectable solutions.

The compositions may also include diluents of various buffer content (*e.g.*, Tris-HCl, acetate, phosphate), pH and ionic strength; additives such as solubilizing agents, anti-oxidants, and preservatives. *See, e.g.,* Martin, Remington's Pharmaceutical Sciences, 18th Ed. (1990, Mack Publishing Co., Easton, PA 18042) pages 1435-1712. The compositions may be prepared in liquid form, or may be in dried powder, such as lyophilized form.

After composition, the microparticle composition may be incorporated into a sterile container, which is then sealed and stored at a low temperature, for example 4°C, or it may be freeze-dried. Lyophilisation permits long-term storage in a stabilised form.

Compositions of preferred embodiment of the present invention are not limited to only contain microparticles in a fluid medium. In addition, they may contain other bioactive, chemical, radioactive or like compounds. Where the composition is employed in the treatment of a biological disease state like cancer, it may contain microparticles as described herein and one or more of the following: other microparticles with cytotoxic and/or radioactive effects; therapeutic and/or pharmaceutical compounds; polypeptides; polynucleotides; or chemical compounds that do not fall within the class of therapeutic or pharmaceutical compounds.

Microparticles described herein have been generally illustrated as containing only nanomagnetic particles dispersed within a matrix. In a highly specialized form of the invention the microparticles prepared according to the invention may also be adapted to accommodate chemical, cytotoxic and radioactive agents. Thus, if the microparticles were used to treat diseased tissue they may be capable of delivering heating effects when in the presence of an AC magnetic field and be capable of delivering ionizing radiation or chemotherapeutic drugs. By way of example, such microparticles may be prepared by creating voids within the capsule capable of receiving and storing the radioactive or therapeutic agent.

Methods for producing voids within microparticles are well known in the art and are something that a person of ordinary skill in the field will be familiar with. Alternatively, the radioactive or therapeutic agent may be attached to the surface of the microparticles, again using methods that are well known in the art.

Furthermore, there is disclosed a method for heating a target site in a patient including the steps of:

(i) administering a microparticle composition(s) as herein described to a target site in said patient; and
(ii) exposing the target site to an AC magnetic field of a clinically acceptable frequency and strength (the applicable field conditions), which is capable of inducing heating of the target site.

Preferably, the microparticles are of a size and density that facilitates the effective transport to ultimately embolise the capillary beds supplying the target site.

The microparticle composition(s) of the present invention may be administered to a target site using any suitable technique known in the art. Preferably, the microparticles are administered in such a way as to cause them to concentrate in a target site. For example, the microparticles of the present invention may be administered via intratumoral, peritumoral, or intravascular, intravenous, intraperitoneal, subcutaneous, intrahecal injection or superficial applications. Preferably, the microparticle composition(s) are delivered to the target site via the arterial or venous blood supply.

Microparticle composition(s) of the present invention maximise the heat generating capabilities of the encapsulated nanomagnetic particles upon exposure to an AC magnetic field of appropriate strength and frequency. The affect of the microparticle composition(s) of the present invention can be optimized for maximum heat generation using an AC magnetic field with frequency in the range of about 50-300 kHz and strength of about 60-120 Oe. In a highly preferred embodiment, the operating conditions of the AC magnetic field is a frequency of about 100 kHz and a strength of about 90 Oe.

Subsequent exposure of the target site to an AC magnetic field of appropriate frequency and strength causes the microparticles to heat and this heat is conducted into the immediately surrounding diseased tissue. This method of treatment is known as Selectively Targeted Hyperthermia (STH).

Adequate heating of the target site is required for the use of induced hyperthermia to be effective. Thus, the method provides a means to increase temperature in a target site to above 41 °C. For use on the treatment of diseased tissue, the result is to decrease the viability of malignant cells. A decrease in the viability of malignant cells results in either cell death or increased cell sensitivity to the effects of ionising radiation or chemotherapeutic drugs.

Therefore, it is preferable for the microparticle composition(s) to achieve adequate heating of 42°C for at least 30 minutes. The level of heating induced by the implanted microparticles depends on several factors, including the VAR of the microparticles, the amount of material that can be localised in and around the target site, and the cooling factors in the environment of the microparticle composition(s), such as blood perfusion.

However, it is generally accepted that the concentration of microparticle composition(s) throughout the target site volume should be no more than 1.0 % (v/v), preferably no more than 0.5% (v/v) and more preferably no more than 0.25% (v/v). For example, there should preferably be no more than about 0.01 cm$^3$ of microparticle composition(s) per cm$^3$ of tissue. The lower the concentration of microparticle composition(s) required to achieve adequate heating the better. If higher concentrations of material are required then it is unlikely that certain very advantageous targeted delivery techniques can be used. Further, in broad terms, deposition of heat at a rate of approximately 100 mW per cm$^3$ of tissue is usually considered adequate to heat said tissue. This heat must be contributed by the administered microparticles. Therefore, a minimum requirement is that the administered microparticles must have a VAR of at least about 10 Watts per cm$^3$ of their volume (10 W/cm$^3$), but more preferably 40 W/cm$^3$ subject to appropriate field conditions.

The effective VAR level must be maintained after the microparticle composition(s) have been administered to the patient and have come in contact with biological fluids. The problems of particle aggregation, sensitivity to surface conditions and particle interaction effects commonly lead to degraded VAR characteristics of superparamagnetic particles outside the very controlled conditions of the laboratory.

**Brief Description of the Drawings**

In the drawings:

Figure 1 shows the temperature of a sample of nanomagnetic particles dispersed in an agar gel as a function of time exposed to an AC magnetic field of strengths 60, 90 and 120 Oe and with frequency 285 kHz.

Figure 2 is a plot of the VAR (in Watts/cm$^3$) calculated for the nanomagnetic particles at the three different magnetic field strengths used.

Figure 3 shows the temperature of a sample of microparticles dispersed in an agar gel as a function of time exposed to an AC magnetic field of strengths 60, 90 and 120 Oe and with frequency 285 kHz.

Figure 4 is a plot of the VAR (in Watts/cm$^3$) calculated for the microparticles at the three different magnetic field strengths used.

Figure 5(a) is a comparison of the sizes of 10 tumours before (black columns) and 14 days after (white columns) being treated by heat from arterially administered microparticles.

Figure 5(b) is a comparison of the sizes of 10 tumours, before (black columns) and 14 days after (white columns) being treated by heat from unencapsulated nanomagnetic particles injected directly into the tumour milieu.

Figure 6 is a graph showing the heat output (W/g$_\gamma$) from nanomagnetic particles as the concentration (vol%) of the nanomagnetic particles increase

## Best Mode(s) for Carrying Out the Invention

## Example 1: VAR of Magnetite Nanoparticles

Magnetite nanoparticles (nanomagnetic particles) were produced using a water-in-oil microemulsion formed using a surfactant, co-surfactant, oil phase and an aqueous phase. The particle size is determined by the water to surfactant ratio. Two solutions, one containing $FeSO_4$ (aq) and the other $NH_3$ (aq), with an equal water to surfactant ratio are mixed for 3 hours. The oil phase and water are removed by drying in an oven overnight. The resultant particles are then washed to remove residual surfactant.

The VAR of the magnetite nanoparticles was determined using the following method:

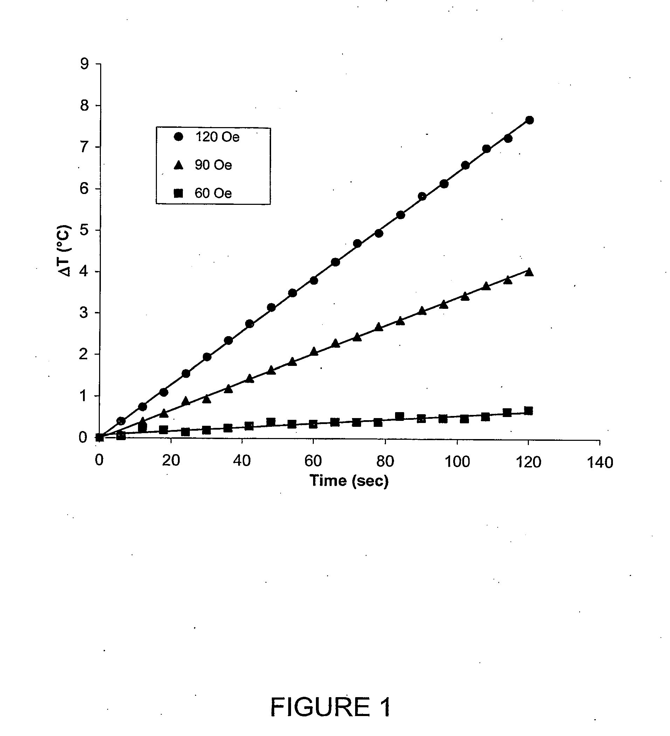

100 mg of magnetite nanoparticles were dispersed and fixed in 10 g of agar gel in a glass vial. The glass vial was then supported in the middle of a coil that forms part of a circuit to generate an AC magnetic field at a frequency of 285 kHz. Fibre-optic thermometry probes were inserted into the gel. The AC magnetic field was adjusted to 60 Oe and the temperature of the gel/nanoparticle mixture recorded as a function of time for at least 2 minutes. The gel sample was then allowed to cool back to room temperature and the process repeated at 90 Oe and then again at 120 Oe. Figure 1 shows the recorded temperature curves for the magnetite impregnated gel sample measured at each of the field values.

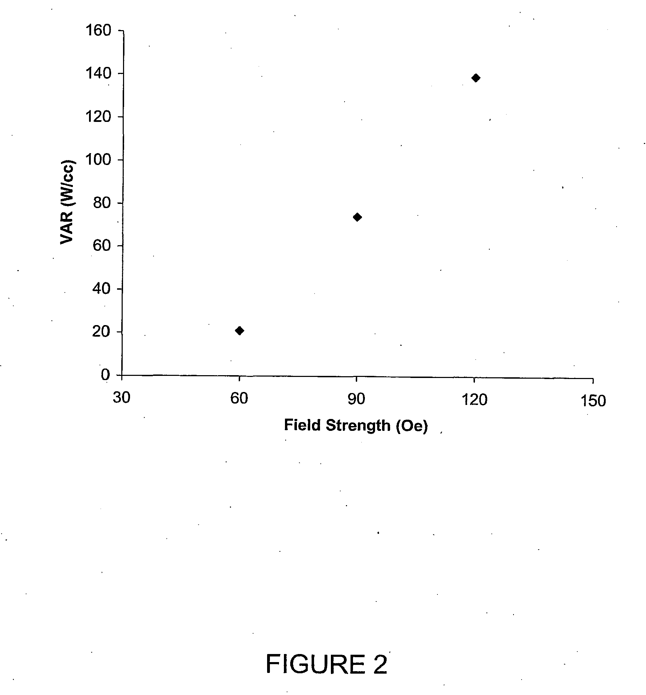

From the measured heating rate it is possible to calculate the VAR of the magnetite nanoparticles. The values calculated for these magnetite nanoparticles at 60, 90 and 120 Oe and 285 kHz are 21 W/cm$^3$, 74 W/cm$^3$ and 139 W/cm$^3$ respectively (data plotted in figure 2).

## Example 2: Formation of Microparticles Incorporating Nanomagnetic Particles

Approximately 0.6 g of magnetite nanoparticles described in Example 1 were dispersed in 6 ml of Elast-Eon™ dissolved in 4-methyl-2-pentanone (7.5% w/v). The mixture was dispersed using a Branson Model 450 Sonifier on power setting 2 for 5 minutes using the 1/8 inch tapered microtip. The mixture was then dropped into a beaker containing 130 ml of 0.25% (wt/v) poly-vinyl alcohol (2.5 g of PVA 87-89% hydrolyzed, MW 124,000-186,000 dissolved in 1 Litre of water) while being mixed with a homogenising mixer set at 2,500 rpm. The mixture was then left mixing for 10 minutes after which it was left to mix very slowly for at least 60 minutes to allow all the 4-methyl-2-pentanone to evaporate. The mixture was then sieved to select microparticles in the size range 25-45 micron. This whole process was repeated 3 times to obtain a quantity of microparticles.

The average density of these particles was then determined to be 2.7 g/cm$^3$ which is within the range required for clinical application. This average density corresponds to approximately 40% of the total microparticle volume or 75% of the total microparticle weight being magnetite nanoparticles.

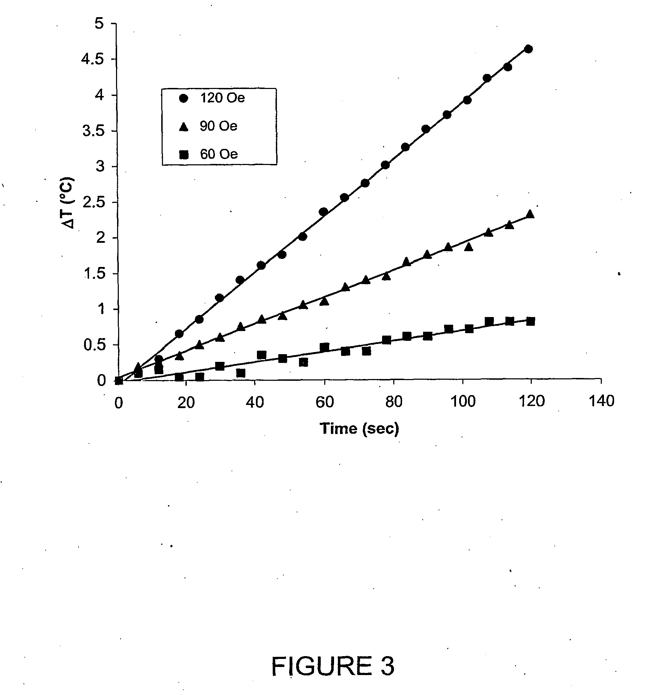

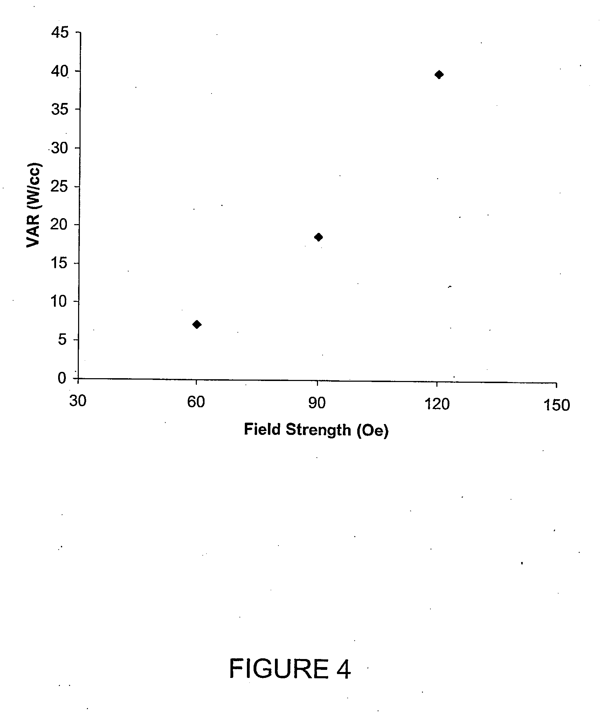

Using the method described in Example 1, the VAR of these microparticles was determined to be 40.0 W/cm$^3$ as is required for clinical use. The heating curve is shown in Figure 3. Also shown are the curves measured using applied fields of 60 and 90 Oe, with the corresponding VARs being 7.2 W/cm$^3$ and 18.7 W/cm$^3$ respectively. The VAR inferred from the temperature rise data for the microparticles is shown plotted as a function of field strength in figure 4.

## Example 3: Demonstration of the Therapeutic Superiority of Arterially Administered Magnetic Microparticles

Objective: It is possible to arterially administer microparticles comprising nanomagnetic particles and a matrix or to directly inject nanomagnetic particles into liver tumours in animal models to generate heating upon exposure to an alternating magnetic field. It was the objective of this study to compare the response of liver tumours to heating from arterially administered microparticles containing nanomagnetic particles and a matrix versus the case when tumours are heated from unencapsulated nanomagnetic particles injected directly into the tumour milieu.

Method: Ten rabbits containing implanted hepatic VX2 carcinomas received a hepatic arterial infusion of microparticles, comprising nanomagnetic particles and a matrix, while the tumours of another ten rabbits were directly injected with the unencapsulated nanomagnetic particles, i.e. nanomagnetic particles not incorporated into a matrix. It was found to be necessary to mix the directly injected nanomagnetic particles with lipiodol and histoacryl to form a viscous emulsion. Histoacryl is a tissue adhesive that remains fluid at room temperature for approximately twenty minutes, but sets fast on contact with ionic fluid. This was used to prevent regurgitation of nanomagnetic particles back up the injection track in the tumour. The tumours of both groups were then heated to over 42°C for twenty minutes by application of an AC magnetic field. Fourteen days after treatment the tumour response was assessed by comparison of tumour volumes before and after treatment and by comparison of the post treatment tumour mass with the mass of untreated control tumours of the same age. The tumours were also analysed histologically.

Results: All tumours treated with arterially administered microparticles decreased in volume by 50-94% (p=0.005) and their average mass (median=1.73g, N=10) was significantly less than that of untreated control tumours (median=8.01g, N=20) (p<0.001). Three of the treated tumours were completely necrotic, while the remainder were at least 80% necrotic. Nine of the ten tumours treated by directly injected nanomagnetic particles increased in volume by at least 143% (p=0.01) and their average mass (median=5.68g, N=10) was not significantly different to that of the untreated control tumours (p=0.56).

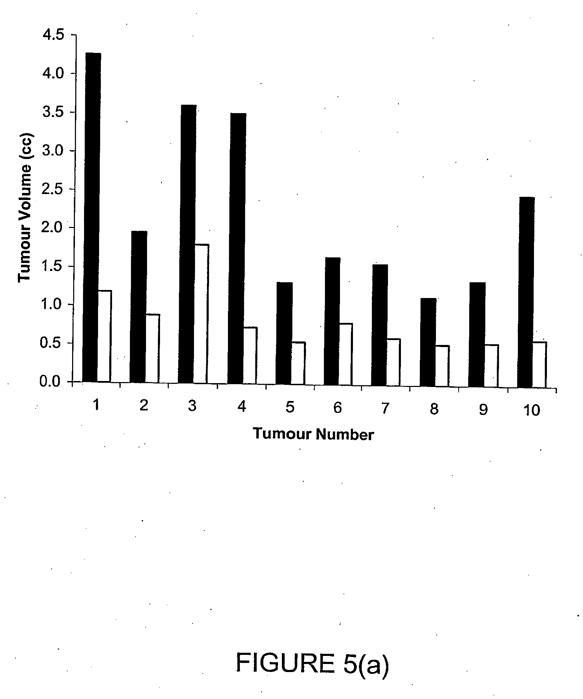

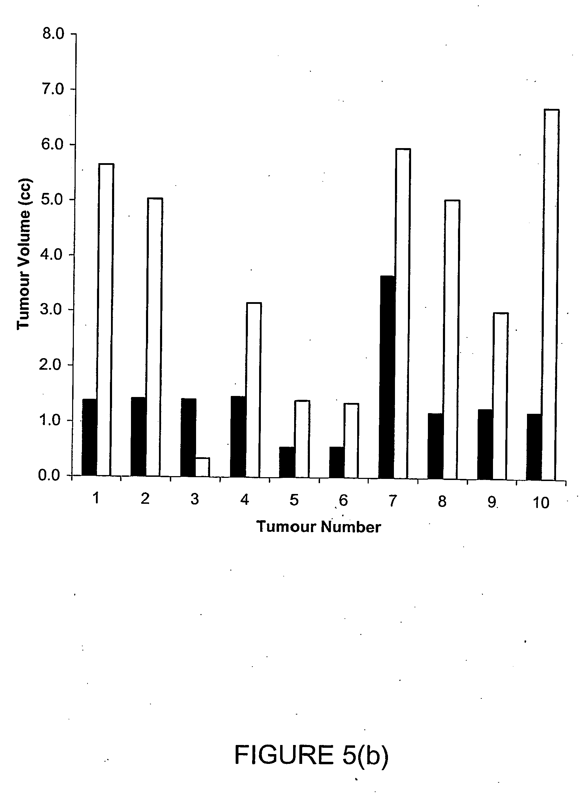

Figures 5 (a) and (b) show each individual tumour size before (black columns) and 14 days after (white columns) a single heat treatment either by arterially administered microparticles comprising nanomagnetic particles and a matrix (figure 5(a)) or by directly injected nanomagnetic particles (figure 5(b)). The superiority of treatment by arterial microparticles is clear from this data.

Conclusion: This example teaches that the therapeutic benefit of heating via arterially administered microparticles is much more effective than heating by nanomagnetic particles injected directly into the tumour. This is probably due to the more widespread particle distribution that can be achieved using arterial administration, which results in a more even tumour treatment. The nature of direct injection can lead to the under-treatment of liver tumours.

Note that in this case the microparticles and the magnetic field conditions used differ from those claimed in this patent. Never the less, the example does serve to illustrate the relative merits of the arterially administered microparticles approach to treatment of cancer.

## Example 4: Effect of Nanomagnetic Particle Concentration

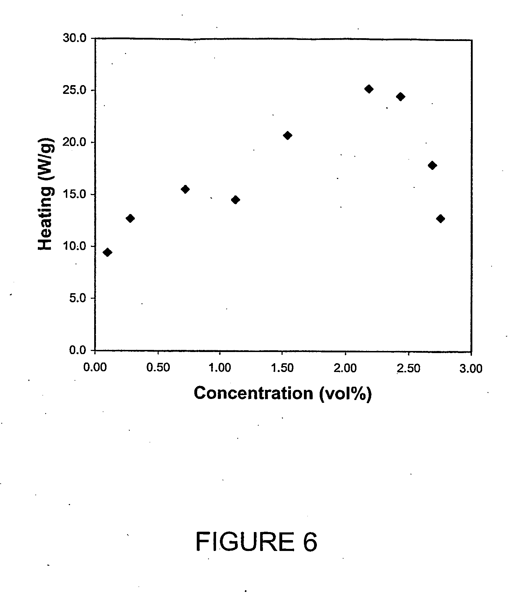

The effect of mutual magnetic interactions between adjacent nanomagnetic particles is not clearly understood. Therefore, we measured the heat output from samples of aqueous suspensions of maghemite nanoparticles of various concentrations, i.e. of various average interparticle spacing. The data in Figure 6 shows that the heat output from the nanomagnetic particles, in terms of watts per gram of maghemite ($W/g_\gamma$), increases as concentration increases until the point where aggregation occurs. In this particular sample, aggregation occurred at about 2 vol% and the measured $W/g_\gamma$ is substantially degraded thereafter.

This data highlights two important factors: (i) the importance of synthesising microparticles in which the incorporated nanomagnetic particles are dispersed rather than being allowed to form aggregated clumps; and (ii) the added benefits obtained by being able to incorporate a high particle loading (vol%) into the microparticles.

Up to approximately 30% of the total volume of each microparticle needs to be occupied by nanomagnetic particles in order to get enough heating from each microparticle. This is a technologically difficult task. However, this data suggests that, since the $W/g_\gamma$ increases as the concentration increases, it may be possible to reduce the nanomagnetic particle loading in the microparticles and still achieve the required heating outcome. The heating characteristics of solid microparticles will be far more stable and less prone to changes due to environmental factors than would be a magnetic fluid containing coated nanomagnetic particles.

## Claims

1. A microparticle composition comprising nanomagnetic particles and a matrix, wherein the nanomagnetic particles are magnetic nanoparticles that are distributed within the matrix and wherein the composition has a VAR of at least 1 Watts/cm$^3$ under an alternating magnetic field that has a frequency in the range of 50-300 kHz and a field strength of 60-120 Oe; and the size of the microparticles in the composition range from 100 nm to 200 microns.

2. A microparticle composition according to claim 1 wherein the size range of the microparticles in the composition is between 25 microns to 45 microns.

3. A microparticle composition according to claim 1, wherein less than 40% of the volumetric loading of the microparticle composition is magnetic nanoparticles.

4. A microparticle composition according to claim 2 wherein the volumetric loading of nanomagnetic particles is less than 30% of the microparticle composition.

5. A microparticle composition according to claim 2 wherein the volumetric loading of nanomagnetic particles is less than 20% of the microparticle composition.

6. A microparticle composition according to claim 2 wherein the volumetric loading of nanomagnetic particles is less than 15% of the microparticle composition.

7. A microparticle composition according to claim 2 wherein the volumetric loading of nanomagnetic particles is less than 10% of the microparticle composition.

8. A microparticle composition according to claim 2 wherein the volumetric loading of nanomagnetic particles is less than 5% of the microparticle composition.

**9.** A microparticle composition according to claim 1 wherein the alternating magnetic field has a frequency in the range of about 100 kHz and field strength of about 90 Oe.

**10.** A microparticle composition according to anyone of claims 1 to 9 wherein the nanomagnetic particles are superparamagnetic particles.

**11.** A microparticle composition according to claim 10 wherein the superparamagnetic particles are either: (a) ferrites of general formula $MO.Fe_2O_3$ where M is a bivalent metal selected from Fe, Co, Ni, Mn, Be, Mg, Ca, Ba, Sr, Cu, Zn, Pt or mixtures thereof; or (b) magnetoplumbite type oxides of the general formula $MO.6Fe_2O_3$ where M is a large bivalent ion, metallic iron, cobalt or nickel.

**12.** A microparticle composition according to claim 11 wherein the superparamagnetic particles are free Fe, Ni, Cr or Co; oxides of Fe, Ni, Cr or Co; or mixtures of Fe, Ni, Cr or Co.

**13.** A microparticle composition according to claim 11 wherein the superparamagnetic particles is a nanoparticle of iron oxide selected from the group of magnetite ($Fe_3O_4$) or maghemite ($\gamma$-$Fe_2O_3$) and having a particle size of less than 50nm.

**14.** A microparticle composition according to claim 12 wherein the superparamagnetic particles are maghemite nanoparticles.

**15.** A microparticle composition according to anyone of claims 10 to 14 wherein the superparamagnetic particles have a size of between 1 nm and 40nm.

**16.** A microparticle composition according to any one of claims 1 to 15 wherein the matrix is a polymer matrix.

**17.** A microparticle composition according claim 16 wherein the polymer matrix is a biocompatible material.

**18.** A microparticle composition according to either claims 16 or 17 wherein the polymer matrix is a biodegradable polymer matrix.

**19.** A microparticle composition according to anyone of claims 1 to 18 wherein the nanomagnetic particles in the composition are adapted for site specific delivery to or accumulation within a tissue in a patient.

**20.** A microparticle composition according to anyone of claims 1 to 19 wherein the composition includes pharmaceutically acceptable carriers and or diluents.

**21.** A microparticle composition according to anyone of claims 1 to 20 wherein the composition includes bioactive, chemical or radioactive agents that are capable of delivering a therapeutic effect to a patient.

**22.** A method for heating a non-living object including the steps of:

(i) inserting a microparticle composition according to anyone of claims 1 to 21 into the object; and
(ii) exposing the object to an alternating magnetic field,
wherein the combination of the alternating magnetic field with the microparticle composition induces heat within the object.

**23.** The use of a microparticle composition according to any one of claims 1 to 21 for the manufacture of a medicament for the treatment of tumours.

**Patentansprüche**

**1.** Mikroteilchenzusammensetzung, die nanomagnetische Teilchen und Matrix umfasst, wobei die nanomagnetischen Teilchen magnetische Nanoteilchen sind, die in der Matrix vorteilt sind, und wobei die Zusammensetzung eine VAR (volumetrische Absorptionsrate) von wenigstens 1 W/cm³ aufweist, in einem alternierenden magnetischen Feld, das eine Frequenz im Bereich von 50-300 kHz und eine Feldstärke von 60-120 Oe hat, und die Größe der Mikroteilchen in der Zusammensetzung von 100 nm bis 200 Mikrometer reicht.

**2.** Mikroteilchenzusammensetzung gemäß Anspruch 1, wobei der Größenbereich der Mikroteilchen in der Zusammensetzung zwischen 25 Mikrometer bis 45 Mikrometer liegt

**3.** Mikroteilchenzusammensetzung gemäß Anspruch 1, wobei weniger als 40% der volumetrischen Beschickungsmenge der Mikroteilchenzusammensetzung aus magnetischen Nanoteilchen besteht.

**4.** Mikroteilchenzusammensetzung gemäß Anspruch 2, wobei die volumetrische Beschickungsmenge an nanomagnetischen Teilchen weniger als 30% der Mikroteilchenzusammensetzung beträgt.

**5.** Mikroteilchenzusammensetzung gemäß Anspruch 2, wobei die volumetrische Beschickungsmenge an nanomagnetischen Teilchen weniger als 20% der Mikroteilchenzusammensetzung beträgt.

**6.** Mikroteilchenzusammensetzung gemäß Anspruch 2, wobei die volumetrische Beschickungsmenge an nanomagnetischen Teilchen weniger als 15% der Mikroteilchenzusammensetzung beträgt.

**7.** Mikroteilchenzusammensetzung gemäß Anspruch 2, wobei die volumetrische Beschickungsmenge an nanomagnetischen Teilchen weniger als 10% der Mikroteilchenzusammensetzung beträgt.

**8.** Mikroteilchenzusammensetzung gemäß Anspruch 2, wobei die volumetrische Beschickungsmenge an nanomagnetischen Teilchen weniger als 5% der Mikroteilchenzusammensetzung beträgt.

**9.** Mikroteilchenzusammensetzung gemäß Anspruch 1, wobei das alternierende magnetische Feld eine Frequenz im Bereich von ungefähr 100 kHz und eine Feldstärke von ungefähr 90 Oe hat.

**10.** Mirkoteilchenzusammensetzung gemäß irgendeinem der Ansprüche 1 bis 9, wobei die nanomagnetischen Teilchen superparamagnetischen Teilchen sind.

**11.** Mikroteilchenzusammensetzung gemäß Anspruch 10, wobei die superparamagnetischen Teilchen entweder (a) Ferrite der allgemeinen Formel $MO.Fe_2O_3$ sind, wobei M ein bivalentes Metall ist, ausgewählt unter Fe, Co, Ni, Mn, Be, Mg, Ca, Ba, Sr, Cu, Zn, Pt oder Gemischen davon, oder (b) Oxide vom Magnetoplumbit-Typ mit der allgemeinen Formel $MO.6Fe_2O_3$ sind, wobei M ein großes bivalentes Ion, metallisches Eisen, Kobalt oder Nickel ist.

**12.** Mikroteilchenzusammensetzung gemäß Anspruch 11, wobei die superparamagnetischen frei von Fe, Ni, Cr oder Co, Oxiden von Fe, Ni, Cr oder Co oder Gemischen von Fe, Ni, Cr oder Co sind.

**13.** Mikroteilchenzusammensetzung gemäß Anspruch 11, wobei die superparamagnetischen Teilchen Nanoteilchen aus Eisenoxid sind, ausgewählt aus der Gruppe von Magnetit ($Fe_3O_4$) oder Maghemit ($\gamma$-$Fe_2O_3$), und eine Teilchengröße von weniger als 50 nm haben.

**14.** Mikroteilchenzusammensetzung gemäß Anspruch 12, wobei die superparamagnetischen Teilchen Maghemit-Nanoteilchen sind.

**15.** Mikroteilchenzusammensetzung gemäß irgendeinem der Ansprüche 10 bis 14, wobei die superparamagnetischen Teilchen eine Größe zwischen 1 nm und 40 nm aufweisen.

**16.** Mikroteilchenzusammensetzung gemäß irgendeinem der Ansprüche 1 bis 15, wobei die Matrix eine Polymermatrix ist.

**17.** Mikroteilchenzusammensetzung gemäß Anspruch 16, wobei die Polymermatrix ein biokompatibles Material ist.

**18.** Mikroteilchenzusammensetzung gemäß entweder Anspruch 16 oder 17, wobei die Polymermatrix eine biologisch abbaubare Polymermatrix ist.

**19.** Mikroteilchenzusammensetzung gemäß irgendeinem der Ansprüche 1 bis 18, wobei die nanomagnetischen Teilchen in der Zusammensetzung für eine ortsspezifische Abgabe oder Anreicherung in einem Gewebe in einem Patienten angepasst sind.

**20.** Mikroteilchenzusammensetzung gemäß irgendeinem der Ansprüche 1 bis 19, wobei die Zusammensetzung pharmazeutisch verträgliche Träger oder Verdünnungsmitteil einschließt.

**21.** Mikroteilchenzusammensetzung gemäß irgendeinem der Ansprüche 1 bis 20, wobei die Zusammensetzung bioaktive, chemische oder radioaktive Mittel umfasst, die in der Lage sind, eine therapeutische Wirkung an einen Patienten abzugeben.

**22.** Verfahren zum Erwärmen eines nichtlebenden Objekts, das die Schritte umfasst, bei denen man

(i) eine Miroteilchenzusammensetzung gemäß irgendeinem der Ansprüche 1 bis 21 in das Objekt einbringt und
(ii) das Objekt einem alternierenden magnetischen Feld aussetzt,
wobei die Kombination des alternierenden magnetischen Felds mit der Mikroteilchenzusammensetzung in dem Objekt eine Erwärmung hervorruft.

**23.** Verwendung einer Mikroteilchenzusammensetzung gemäß irgendeinem der Ansprüche 1 bis 21 für die Herstellung eines Medikaments zur Behandlung von Tumoren.

**Revendications**

**1.** Composition de microparticules comprenant des particules nanomagnétiques et une matrice, dans laquelle les particules nanomagnétiques sont des nanoparticules magnétiques qui sont réparties dans la matrice et dans laquelle la composition possède une VAR d'au moins 1 Watt/cm$^3$ dans une champ magnétique alternatif qui présenté une fréquence de 50 à 300 kHz et une intensité de champ de 60 à 120 Oe ; et la taille des microparticules de la composition est de 100 nm 200 micromètres.

**2.** Composition de microparticules selon la revendication 1, dans laquelle la plage des tailles des microparticules de la composition est comprise entre 25 micromètres et 45 micromètres.

**3.** Composition de microparticules selon la revendication 1, dans laquelle moins de 40 % de la charge volumétrique de la composition de microparticules sont des nanoparticules magnétiques.

**4.** Composition de microparticules selon la revendication 2, dans laquelle la charge volumétrique des particules nanomagnétiques est inférieure à 30 % de la composition de microparticules.

**5.** Composition de microparticules selon la revendication 2, dans laquelle la charge volumétrique des particules nanomagnétiques est inférieurs à 20 % de la composition de microparticules.

**6.** Composition de microparticules selon la revendication 2, dans laquelle la charge volumétrique des particules nanomagnétiques est inférieure à 15 % de la composition de microparticules.

**7.** Composition de microparticules selon la revendication 2, dans laquelle la charge volumétrique des particules nanomagnétiques est inférieure à 10 % de la composition de microparticules.

**8.** Composition de microparticules selon la revendication 2, dans laquelle la charge volumétrique des particules nanomagnétiques est inférieure à 5 % de la composition de microparticules.

**9.** Composition de microparticules selon la revendication 1, dans laquelle le champ magnétique alternatif possède une fréquence d'environ 100 kHz et une intensité de champ d'environ 90 Oe.

**10.** Composition de microparticules selon l'une quelconque des revendications 1 à 9, dans laquelle les particules nanomagnétiques sont des particules superparamagnétiques.

**11.** Composition de microparticules selon la revendication 10, dans laquelle les particules superparamagnétiques sont soit (a) des ferrites de formule générale $MO.Fe_2O_3$ où M est un métal bivalent choisi parmi Fe, Co, Ni, Mn,Be, Mg, Ca, Ba, Sr, Cu, Zn, Pt ou des mélanges de ceux-ci ; soit (b) des oxydes de type magnétoplombite de formule générale $MO.6Fe_2O_3$ où M est un grand ion bivalent, du fer métallique, du cobalt ou du nickel.

**12.** Composition de microparticules selon la revendication 11, dans laquelle les particules superparamagnétiques sont des ions libres de Fe, Ni, Cr ou Co ; des oxydes de Fe, Ni, Cr ou Co ; ou des mélanges de Fe, Ni, Cr ou Co.

**13.** Composition de microparticules selon la revendication 11, dans laquelle les particules superparamagnétiques sont une nanoparticule d'oxyde de fer choisie dans le groupe de magnétite ($Fe_3O_4$) ou de maghémite ($\gamma$-$Fe_2O_3$) et ont une taille de particule inférieure 50 nm.

**14.** Composition de microparticules selon la revendication 12, dans laquelle les particules superparamagnétiques sont des nanoparticules de maghémite.

**15.** Composition de microparticules selon l'une quelconque des revendications 10 à 14, dans laquelle les particules superparamagnétiques ont une taille comprise entre 1 nm et 40 nm.

**16.** Composition de microparticules selon l'une quelconque des revendications 1 à 15, dans laquelle la matrice est une matrice polymère.

**17.** Composition de microparticules selon la revendication 16, dans laquelle la matrice polymère est un matériau biocompatible.

**18.** Composition de microparticules selon l'une quelconque des revendications 16 ou 17, dans laquelle la matrice polymère est une matrice polymère biodégradable.

**19.** Composition de microparticules selon l'une quelconque des revendications 1 à 18, dans laquelle les particules nanomagnétiques de la composition sont conçues par une libération spécifique à une site dans un tissu d'un patient ou pour une accumulation dans ledit tissu.

**20.** Composition de microparticules selon l'une quelconque des revendications 1 à 19, dans laquelle la composition comprend des charges pharmaceutiquement acceptables et/ou des diluants.

**21.** Composition de microparticules selon l'une quelconque des revendications 1 à 20, dans laquelle la composition comprend des agents bioactifs, chimiques ou radioactifs qui sont capables d'avoir un effet thérapeutique sur une patient.

**22.** Procédé permettant de chauffer un objet non vivant, comprenant les étapes consistant à :

(i) insérer une composition de microparticules selon l'une quelconque des revendications 1 à 21 dans l'objet ; et
(ii) exposer l'objet à un champ magnétique alternatif,

dans lequel l'association du clamp magnétique alternatif et de la composition de microparticules induit de la chaleur à l'intérieur de l'objet.

**23.** Utilisation d'une composition de microparticules selon l'une quelconque des revendications 1 à 21 pour la fabrication d'un médicament destiné au traitement de tumeurs.

FIGURE 1

FIGURE 2

FIGURE 3

FIGURE 4

FIGURE 5(a)

FIGURE 5(b)

FIGURE 6

**EP 1 594 571 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 0913167 A

**Non-patent literature cited in the description**

- **MARTIN.** Remington's Pharmaceutical Sciences. Mack Publishing Co, 1990
- **MARTIN.** Remington's Pharmaceutical Sciences. Mack Publishing Co, 1990, 1435-1712